# EUROPÄISCHE PATENTSCHRIFT

(11) **EP 1 538 997 B1**
(45) Veröffentlichungstag und Bekanntmachung des Hinweises auf die Patenterteilung: **06.05.2009**
(21) Anmeldenummer: 02764773.4
(22) Anmeldetag: 24.07.2002
(51) Int. Cl.: A61B 17/12

(54) **VORRICHTUNG ZUR IMPLANTATION VON OCCLUSIONSWENDELN**
DEVICE FOR THE IMPLANTATION OF OCCLUSION COILS
DISPOSITIF POUR IMPLANTER DES HELICES D'OCCLUSION

(43) Veröffentlichungstag der Anmeldung: 15.06.2005
(73) Patentinhaber: Dendron GmbH, 44799 Bochum (DE)
(72) Erfinder: MONSTADT, Hermann, 44797 Bochum (DE); HENKES, Hans, 44797 Bochum (DE)
(74) Vertreter: Schneiders, Josef
(86) Internationale Anmeldenummer: PCT/EP2002/008241
(87) Internationale Veröffentlichungsnummer: WO 2004/014239

(56) Entgegenhaltungen:
- EP-A- 0 792 623
- WO-A-00/21443
- WO-A-01/32085
- WO-A-97/42881
- WO-A-99/09894
- US-A- 5 800 454

## Beschreibung

Die Erfindung betrifft eine Occlusionswendel sowie ein medizinisches Implantat, das zur Deponierung in zu verschließenden Körperhohlräumen oder Blutgefäßen bestimmt ist. Die Erfindung bezieht sich weiterhin auf eine Vorrichtung zur Implantation von Occlusionswendeln in Körperhohlräumen oder Blutgefäßen mit einem Katheter, einer in dem Katheter in Längsrichtung verschiebbaren Occlusionswendel und mindestens einem in der Occlusionswendel befestigten, als flexibles Längenelement ausgebildeten Sicherungsmittel, welches die Länge der Occlusionswendel zumindest in einem Teilbereich durchläuft.

Der Einsatz endovaskulärer Techniken zur Occlusion von Körperhohlräumen oder Gefäßen wie Arterien, Venen, Eileitern oder vaskulären Fehlbildungen (z. B. vaskulärer Aneurysmen) ist bekannter Stand der Technik. Die Occlusionswendel wird dabei in der Regel mit Hilfe eines endovaskulären Führungsdrahtes durch einen Katheter in den zu occludierenden Hohlraum eingeführt und deponiert.

Im Vorfeld der Deponierung werden die zu implantierenden Occlusionswendeln mit Hilfe des Katheters durch das Blutgefäßsystem gesteuert und am Zielort aus dem Katheter in das zu occludierende Arial vorgeschoben. Im Idealfalle schließt sich die Abtrennung der Wendel an. Im Falle fehlerhafter Positionierung oder einer für den zu occludierenden Bereich zu groß dimensionierten Occlusionswendel, muß diese jedoch repositioniert oder vollständig in den Katheter zurückgezogen werden, um anschließend eine korrekte Positionierung oder die Einbringung einer korrekt dimensionierten Occlusionswendel zu gestatten. Derartige Manöver im Gefäßsystem bergen die Gefahr, daß Teile der Wendel durch Zug- oder Torsionsbeanspruchung auseinandergezogen und so irreversibel plastisch deformiert werden, reißen oder brechen, was eine lebensgefährliche Embolie nach sich ziehen kann.

Um diese Gefahren zu minimieren, ist es aus der Schrift PCT/US 98/17885 bekannt, ein flexibles Sicherungsmittel in der Occlusionswendel zu befestigen. Der Nachteil dieser Vorrichtung besteht darin, daß das aus einem Polymermaterial bestehende Sicherungsmittel hinsichtlich Biegebeanspruchung und Zugfestigkeit nicht ideal ist. So kann es, bei zu großer Torsionsbelastung und/oder zu starker Zugbelastung auch bei dieser Vorrichtung zu einem Zerreißen der Wendel im Blutgefäßsystem kommen.

Aus der EP 0 792 623 A1 ist ein Sicherungsmittel bekannt, welches aus einem Polymer oder auch aus einem Metall, beispielsweise einem Formgedächtnismetall bestehen kann. In der WO 97/42881 A1 wird eine Wendel mit einem Kern beschrieben, dessen Biegesteifigkeit entlang der Längsachse variiert und der aus einer Formgedächtnislegierung bestehen kann.

Der Begriff "Formgedächtnis" ist dem angesprochenen Durchschnittsfachmann hinlänglich bekannt, er umfasst sowohl das mechanisch als auch das thermisch induzierte Formgedächtnis. Als Materialien mit Formgedächtniseigenschaften werden im Rahmen dieser Erfindung sowohl Materialien verstanden, die entweder ein thermisches oder ein mechanisches Formgedächtnis aufweisen, wie auch solche Materialien mit thermisch und mechanisch induziertem Formgedächtnis. Als Materialien kommen dabei sowohl organische Werkstoffe als auch metallische Legierungen mit Formgedächtniseigenschaften in Frage.

Solche Materialien weisen dabei die Fähigkeit auf, abhängig von der Temperatur, zwischen einem eher rigiden und einem sehr flexiblen Zustand hin und her zu wechseln, wobei sie auch Übergangszustände durchlaufen. Solche Materialien sind wesentlich stärker durch Biegung oder Zug beanspruchbar als herkömmliche Werkstoffe. Das Material kann dabei insbesondere im flexiblen Zustand extrem stark verbogen und gedehnt werden, ohne zu reißen. Erst bei Erhöhung der Temperatur kehrt es in seinen rigiden Zustand zurück, was im Falle einer vorangegangenen Verformung mit einer Formänderung einhergeht. Die jeweilige Temperaturschwelle kann, in für den zuständigen Fachmann allgemein bekannter Weise, durch die Zusammensetzung des Materials gesteuert werden.

Gemäß einer bevorzugten Ausführungsform der Erfindung besteht das Sicherungsmittel im wesentlichen oder vollständig aus dem Material mit Formgedächtniseigenschaften.

Die Ausbildung des Sicherungsmittels mit oder aus Material mit Formgedächtniseigenschaften verleiht der erfindungsgemäßen Vorrichtung eine erhöhte Biegefestigkeit und erhöhte Stabilität gegen Zug- und Torsionsbeanspruchung.

Gemäß einer zweckmäßigen Ausführungsform ist das Sicherungsmittel der erfindungsgemäßen Vorrichtung länger dimensioniert als der Teilbereich der Occlusionswendel, über den es sich erstreckt. Die Längendimensionierung des Sicherungsmittels gewährleistet zudem eine weniger rigide Anordnung trotz stabiler Befestigung in der Occlusionswendel, so daß das Sicherungsmittel in der Occlusionswendel ohne äußere Einwirkung keiner Zugbeanspruchung unterliegt, was eine große Stabilität und Flexibilität gewährleistet.

Es ist zweckmäßig, wenn für die Herstellung des Sicherungsmittels eine metallische Legierung mit Formgedächtniseigenschaften verwendet wird. Dies können Legierungen sein, die entweder die Fähigkeit zur temperaturinduzierten oder spannungsinduzierten martensitischen Transformation aufweisen. Besonders bevorzugt sind jedoch Legierungen mit der Fähigkeit, sowohl eine temperaturinduzierte als auch eine spannungsinduzierte martensitische Transformation zu durchlaufen. Hier eignen sich insbesondere Titan und Nickel enthaltende Legierungen sowie Eisenbasis- oder Kupferbasislegierungen.

Die Verwendung einer solchen Legierung mit Formgedächtniseigenschaften weist zum einen den Vorteil auf, daß Metall/Metall Verbindungen zwischen Occlusionswendel und Sicherungsmittel besser gefertigt werden können als solche mit Kunststoff/Metall Verbindungen. Auf Titan und Nickel basierende Legierungen weisen überdies den Vorteil auf, daß ihre mechanischtechnologischen Eigenschaften gut untersucht sind.

Titan-Nickel Legierungen weisen dabei, abhängig von der Temperatur unterschiedliche Kristallstrukturen auf: Die bei hoher Temperatur vorliegende Phase wird als Austenit bezeichnet. Ihre Atomanordnung ist kubisch flächenzentriert; sie stellt die stabile Phase dar. Bei niedriger Temperatur liegen die Atome einer solchen Legierung in tetragonal verzerrter, kubischraumzentrierter Anordnung vor. Sie wird als Martensit bezeichnet. Die durch die Temperatur bedingte Martensitphase wird auch als temperaturinduzierter Martensit (TIM) bezeichnet. Durch die Wahl der Legierungszusammensetzung kann dabei bestimmt werden, bei welcher Temperatur ein Übergang (Transformation) von der einen zur anderen Phase erfolgt, dies kann über einen Bereich von -100 bis 100°C erfolgen.

Wirkt während der Umwandlung von Austenit in Martensit (infolge einer Senkung der Temperatur unter einen kritischen Wert) keine äußere Kraft ein, ist keine makroskopische Gestaltsänderung zu beobachten. Im martensischen Zustand ist das Bauteil leicht zu verformen, wobei eine Gestaltsänderung von bis zu ca. 8% erreicht werden kann. Solange das Material unter der kritischen Temperaturschwelle (der Umwandlungstemperatur) bleibt, ist die Verformung stabil. Wird der verformte Martensit jedoch erwärmt, stellt sich bei Überschreiten der Umwandlungstemperatur die ursprüngliche Gestalt wieder ein. Dieses, durch unterschiedliche Umgebungstemperaturen gesteuerte Formgedächtnis des temperaturinduzierten Martensits wird auch als thermisches Formgedächtnis (Shape Memory) bezeichnet.

Neben diesem thermischen Formgedächtnis können metallische Legierungen auch ein mechanisches Formgedächtnis (Superelastizität) besitzen, welches der Einnahme einer spannungsinduzierten martensischen Phase (SIM) beruht: In bestimmten Temperaturbereichen, welche für den Fachmann einfach durch die Wahl einer bestimmten Legierungszusammensetzung einstellbar sind, kann der Übergang in die martensische Phase auch mechanisch durch Einwirkung eines äußeren Zwanges induziert werden (spannungsinduzierter Martensit). Auf diese Weise können Dehnungen bis zu 10% erreicht werden. Bleibt das Material bei dieser Temperatur, welche über der Temperaturschwelle der Umwandlung von Martensit zu Austenit liegt, so geht das Material wieder in die austenitische Phase über, es kommt zu einer Rückverformung.

Die thermische Umwandlung von Martensit zu Austenit findet jedoch innerhalb eines Temperaturbereiches, nicht bei Überschreiten eines streng begrenzten Temperaturwertes statt, so daß es Übergangsphasen in der Materialstruktur gibt. Fällt nun die Einwirkung einer mechanischen Spannung auf einen spannungsinduzierten Martensit bei einer Temperatur in diesem Zwischenbereich weg, so kommt es zu einer teilweisen, spannungsbedingten Rückwandlung zu Austenit und somit zu einer teilweisen Rückverformung. Erst bei einem Anstieg der Temperatur kommt es zur vollständigen Umwandlung in die Austenit Phase. In diesem Falle liegt eine Kombination aus spannungsinduzierter und temperaturinduzierter Phasenumwandlung vor.

Aufgrund der besonders hohen Dehnungen, die solche metallischen Legierungen in martensitischem Zustand (d. h. in temperatur- und/oder spannungsinduziertem martensitischen Zustand) ertragen können, wird das zur Fertigung des Sicherungsmittels verwendete Material vorzugsweise so gewählt, daß das Sicherungsmittel im Katheter in Form spannungs- und/oder temperaturinduzierten Martensits vorliegt und bei Einführung in das Blutgefäßsystem bzw. das zu occludierende Aneurysma durch den Wegfall des durch den Katheter ausgeübten Zwanges und/oder den Anstieg der Umgebungstemperatur zumindest teilweise in die Austenit Phase übergeht und das Aneurysma so stabil ausfüllt. Dabei kann es je nach Situation zweckmäßig sein, eine Legierung zu verwenden, die bei Ausführen aus dem Mikrokatheter eine allein spannungs- oder temperaturinduzierte Umwandlung oder eine gemischte Umwandlung durchläuft.

Zur Nutzung des thermisch induzierten Formgedächtnisses im Körper eignen sich insbesondere Legierungen mit einer Umwandlungstemperatur von zwischen +35 bis +38°C. Zur Nutzung des mechanisch induzierten Formgedächtnisses im Körper eignen sich insbesondere Legierungen mit einer Umwandlungstemperatur von zwischen -15° und +38°C und insbesondere -15°C bis 20°C. Die zur Induzierung von Formgedächtniseffekten im Körper besonders geeigneten Umwandlungstemperaturen sind allerdings dem zuständigen Fachmann hinlänglich bekannt.

Gemäß einer zweckmäßigen Ausführungsform ist das Sicherungsmittel als metallischer Draht ausgebildet. Diese Ausführungsform weist den Vorteil auf, daß Draht sehr kostengünstig ist und einfach mit seinen beiden Enden in der Occlusionsspirale befestigt werden kann.

Besonders zweckmäßig ist eine Ausführungsform, bei der das Sicherungsmittel vorgeformt ist. Zweckmäßig ist hier insbesondere die Vorformung des Sicherungsmittels zu einer Feder bzw. Wendel und vorzugsweise einer Spiralfeder oder helikalen Wendel. Diese Ausführungsform weist besondere Zugfestigkeit auf, da das als Feder oder Wendel ausgebildete Sicherungsmittel bei Zugbeanspruchung zunächst elastisch verformt wird, ohne daß eine irreversible Dehnung eintritt. Bei Überschreiten der Grenze der elastischen Verformbarkeit der Feder bzw. Wendel greift dann die große Flexibilität des Materials mit thermischem Formgedächtnis und/oder superelastischen Eigenschaften, so daß eine doppelte Sicherung gegen das Abreißen der Occlusionswendel gegeben ist.

Da die distale Spitze der Occlusionswendel bei der Einbringung in das Blutgefäß besonders großer Beanspruchung unterliegt, ist es zweckmäßig, wenn das Sicherungsmittel sich bis zum distalen Spitzenabschnitt erstreckt und mit diesem fest, das heißt unlösbar verbunden ist. Vom Spitzenabschnitt kann es sich dabei über einen Teilbereich oder über die gesamte Länge der Occlusionswendel nach proximal erstrecken.

Insbesondere in dem Fall, daß das Sicherungsmittel länger ist als der Bereich der Occlusionswendel, über den es sich erstreckt, ist es ohne Einbußen an Beweglichkeit und Flexibilität möglich, daß das Sicherungsmittel sich komplett vom proximalen Ende bis zum distalen Ende der Occlusionswendel erstreckt, selbst bei großer Länge der Occlusionswendel von mehreren 100 mm. Dies ermöglicht die Sicherung der gesamten Occlusionswendel gegen ein Abreißen, ohne daß die Steuerung und Beweglichkeit derselben innerhalb des Katheters oder Blutgefäßes leidet.

Gemäß einer weiteren zweckmäßigen Ausführungsform der erfindungsgemäßen Vorrichtung, verjüngt sich der die Occlusionswendel bildende Draht und /oder es verjüngt sich das Sicherungsmittel zu seinem proximalen und/oder distalen Ende. Die Verjüngung kann dabei z.B. durch Schleifprozesse herbeigeführt werden. Bei Verjüngung des Sicherungsmittels eignet sich insbesondere eine Ausführungsform der erfindungsgemäßen Vorrichtung, in der das Sicherungsmittel als Draht ausgebildet ist. Derartige sich verjüngende Drähte sind der US 5 800 454 sowie der WO 00/21443 A1 zu entnehmen.

Eine Verjüngung nach proximal trägt der Tatsache Rechnung, daß bei Einbringung der letzten Windungen der Occlusionswendel in Aneurysmen das Aneurysma bereits durch die ersten Längen der Occlusionswendel oder Wendeln unter starker Beanspruchung steht. Versuche der Erfinder haben gezeigt, daß eine Verjüngung der proximalen, also zuletzt eingebrachten Bereiche die Gefahr minimiert, daß die Einbringung dieser letzten Abschnitte mit einer Wandungsruptur des Aneurysmas einhergeht. Die Verjüngung des Sicherungsmittels bzw. Drahtes nach distal (also dem zuerst in das Aneurysma eingebrachten Abschnitt) ermöglicht ein minimal traumatisches Vorgehen, daß die Spitze der Occlusionswendel in diesem Falle besonders flexibel ist. Zur Dimensionierung des Drahtes eignen sich beispielsweise Durchmesser zwischen 0,02 bis 0,2 und insbesondere 0,03 bis 0,1 mm. Eine Verjüngung sollte, um noch genug Zugfestigkeit aufzuweisen, maximal einen Drahtdurchmesser von 0,01 mm, vorzugsweise jedoch nicht weniger als 0,03 mm hervorbringen.

Das Sicherungsmittel ist mittelbar über Verbindungsmittel mit der Occlusionswendel verbunden wobei als Verbindungsmittel Mikrowendeln eingesetzt werden, welche mit dem Sicherungsmittel und der Occlusionswendel fest verbunden sind. Diese Ausführungsform ist besonders kostengünstig, da zur Herstellung herkömmliche Occlusionswendeln verwendet werden können, in welche die Kombination aus Sicherungsmittel und mindestens zwei, an den Enden des Sicherungsmittels befestigte Mikrowendeln eingefügt und über gängige Verfahren mit der Occlusionswendel verbunden werden.

Zur Verbindung der Mikrowendeln mit der Occlusionswendel bzw. dem Sicherungsmittel eignen sich dabei dem zuständigen Fachmann hinlänglich bekannte Maßnahmen wie Verschweißen, Verlöten, Verkleben oder mechanische (d. h. kraft- und/oder formschlüssige) Fügeverfahren.

Bei Verwendung organischer Materialien mit Formgedächtniseigenschaften zur Fertigung des Sicherungsmittels, kommt dabei insbesondere die mechanische Verbindung oder ein Verkleben in Frage. Bei Verwendung metallischer Legierungen mit Formgedächtniseigenschaften eignen sich dagegen grundsätzlich alle vorstehend aufgeführten Verbindungsverfahren. Zur Verbindung der Mikrowendeln mit dem Sicherungsmittel sind dabei mechanische Fügeverfahren (d. h. die kraft- und/oder formschlüssige Verbindung) besonders bevorzugt.

Mikrowendeln und Occlusionswendel sind aus demselben Werkstoff gefertigt. Zur Ausbildung der Mikrowendeln und der Occlusionswendeln eignen sich besonders Platin und Platinlegierungen (insbesondere Pt-Ir Legierungen), Materialien, welche bei der Herstellung von Occlusionswendeln aufgrund ihrer geringen Traumatisierung beim Einbringen ins Gefäßsystem vielfältig zur Herstellung von Occlusionswendeln eingesetzt werden. Zudem können die Mikrowendeln bei dieser Ausführungsform durch Verschweißen besonders stabil mit der Occlusionswendel verbunden werden.

Es ist dabei besonders zweckmäßig, wenn das Sicherungsmittel aus einer Titan und Nickel enthaltenden Legierung besteht, da das Verhalten solcher Legierungen im Stand der Technik gut bekannt ist.

Gemäß einer weiteren bevorzugten Ausführungsform ist die Occlusionswendel als Mikrowendel ausgebildet. Insbesondere ist es zweckmäßig, wenn sie zudem zu einer übergeordneten Struktur vorgeformt ist, welche sie nach Ausbringungaus dem Katheter im Aneurysma ausbildet. Diese Ausführungsform ist zur Thrombosierung von Aneurysmen besonders geeignet.

Es kann darüber hinaus zweckmäßig sein, wenn das Sicherungsmittel selbst zu einem zwei oder dreidimensionalem Design vorgeformt ist, vorzugsweise ebenfalls zu helikalen Windungen oder einem korbartigen Design. Bei dieser Ausführungsform unterstützt das Sicherungsmittel die Einnahme eines zwei oder dreidimensionalen Designs durch die Occlusionswendel. Es kann dabei zur Ausbildung eines solchen Designs ausreichend sein, wenn nur das Sicherungsmittel, nicht aber die Occlusionswendel vorgeformt ist, wenn die bis zum Erreichen der vorgeformten Struktur vom Sicherungsmittel ausgeübte Kraft groß genug ist, die Occiusionswendel ebenfalls in die durch das Sicherungsmittel vorgegebene Form zu zwingen.

Es kann dabei besonders zweckmäßig sein, wenn das Sicherungsmittel als Feder bzw. Wendel und insbesondere als Spiralfeder bzw. helikale Wendel ausgebildet ist, die zu einem übergeordneten, zwei oder dreidimensionalem Design verformt ist.

Gemäß einer besonders bevorzugten Ausführungsform der erfindungsgemäßen Vorrichtung ist das Sicherungsmittel vorgeformt und liegt vorzugsweise in dem Katheter zumindest teilweise in einem Zustand spannungsinduzierten Martensits vor. Bei Ausschieben aus dem Katheter nimmt es dann aufgrund einer durch den Wegfall der mechanischen Spannung und/oder die ansteigende Temperatur im Blutstrom das vorgeformte Design ein und bildet z.B. eine dreidimensionale Helix oder Korbstruktur aus.

In einer weiteren zweckmäßigen Ausführungsform weist die Occlusionswendel eine oder mehrere, in Abständen voneinander angeordnete elektrolytisch korrodierbare Stellen auf und kann so in Verbindung mit einem elektrisch isolierenden Katheter und einer Spannungsquelle sowie einer Kathode, im Kontakt mit einer Körperflüssigkeit eine oder mehrere variabel dimensionierbare Längen durch elektrolytische Korrosion ablösen. Die elektrolytische Ablösung von Occlusionswendeln ist dem zuständigen Fachmann hinlänglich bekannt und weist gegenüber anderen, im Stand der Technik bekannten Maßnahmen zur Ablösung von Occlusionswendeln vielfache Vorteile hinsichtlich Praktikabilität, Sicherheit und Kostengünstigkeit auf.

Es ist dabei besonders zweckmäßig, wenn die Occlusionswendel mehrere elektrolytisch korrodierbare Stellen aufweist, wobei in jedem zwischen diesen Stellen gelegenen Segmente Occlusionswendel ein Sicherungsmittel angeordnet ist, welches sich vorzugsweise jeweils von einem zum anderen Ende jedes Segmentes erstreckt. Diese Ausführungsform ermöglicht die Deponierung variabel dimensionierbarer Längen an Occlusionswendeln, wobei gleichzeitig durch die Sicherung jedes einzelnen, zwischen den elektrolytisch korrodierbaren Stellen angeordneten Segmentes, ein Höchstmaß an Sicherung gegen das Abreißen der Occlusionswendel gewährleistet wird. Occlusionswendeln mit mehreren elektrolytisch korrodierbaren Stellen sind grundsätzlich aus der WO 01/32085 A1 bekannt.

Gemäß einer besonders zweckmäßigen Ausführungsform wird jedes Segment der Occlusionswendel von mindestens zwei, vorzugsweise mindestens drei, ineinandergefügten Mikrowendeln unterschiedlichen Durchmessers gebildet, wobei die jeweils proximal bzw. distal an die elektrolytisch korrodierbare Stelle angrenzende Wendel mit dieser fest verbunden ist. Diese Ausführungsform aus Einzelbauteilen ist besonders kostengünstig in der Fertigung. Das Sicherungsmittel ist dabei entweder an einer der Mikrowendeln angebracht oder indirekt, über die fest mit ihm verbundenen (z. B. selbst als Mikrowendeln ausgebildeten) Verbindungsmittel mit diesen befestigt.

Zweckmäßig ist weiterhin eine Ausführungsform, bei der sich proximal an die Occlusionswendel eine als Führungsdraht ausgebildete Einführhilfe anschließt.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln oder der Tumorembolisation durch Thrombosierung bestimmt.

Die Erfindung bezieht sich weiterhin auf eine Occlusionswendel, wie sie vorstehend beschrieben wurde, sowie ein medizinisches Implantat, welches zumindest ein Sicherungsmittel enthaltenden, deponierbaren Teilbereich einer Occlusionswendel gemäß vorstehender Beschreibung umfaßt.

Die Erfindung ist im Anspruch 1 definiert.

Die Erfindung wird nachfolgend beispielhaft anhand der in den Zeichnungen veranschaulichten Ausführungsbeispiele näher erläutert. Es stellen dar:
- Figur 1: den Längsschnitt durch eine erfindungsgemäße Vorrichtung in Seitenansicht und vielfacher Vergrößerung:
- Figur 2: die vertikal Ansicht einer in ein Beerenaneurysma positionierten Occlusionswendel mit dazugehöriger Vorrichtung in vielfacher Vergrößerung.

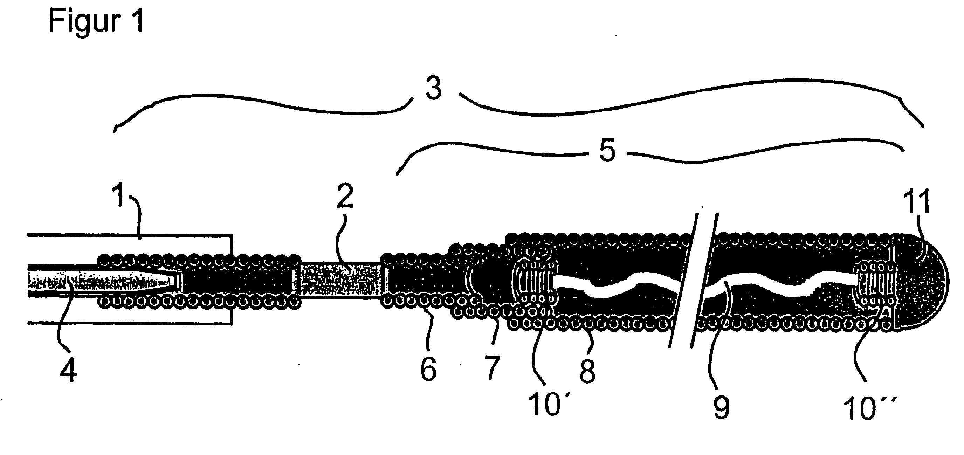

In der Figur 1 ist mit 1 ein elektrisch isolierender Katheter, insbesondere ein biegsam ausgebildeter Mikrokatheter bezeichnet. Durch diesen Mikrokatheter 1 wird die aus einer Platin-Iridium Legierung gefertigte, mit einer elektrolytisch korrodierbaren Stellen 2 aus Edelstahl versehene, als Mikrowendel ausgebildete Occlusionswendel 3 mit Hilfe des schweißtechnisch an die Occlusionswendel 3 gefügten Führungsdrahtes 4 aus dem Mikrokatheter 1 in das Blutgefäßsystem geschoben.

Da die durch artfremdes Schweißen gefügte Verbindung zwischen Führungsdraht 4 und Mikrowendel 3 nicht zur elektrolytischen Ablösung der Mikrowendel 3 bestimmt ist, und folglich nicht von besonders geringem Durchmesser sein muß, ist sie besonders stabil ausgebildet. Die Verwendung von nichtrostendem Edelstahl und einer Platinlegierung für die Ausbildung des Führungsdrahtes einerseits bzw. der Occlusionswendel andererseits ist dabei besonders vorteilhaft, da das im Stahl enthaltene Nickel sich beim Verschweißen zu einer sehr glatten und stabilen Verbindung mit dem Platin fügt.

Die Occlusionswendel 3 weist ein elektrolytisch abtrennbares Segment 5 auf, das mit der proximal davon angeordneten, elektrolytisch korrodierbaren Stelle 2 durch artfremdes Verschweißen verbunden ist. Das Segment weist an seinem proximalen Ende eine erste Mikrowendel 6 mit geringem Durchmesser auf, die an ihrem proximalen Ende schweißtechnisch mit der proximal sich daran anschließenden elektrolytisch korrodierbaren Stelle 2 und an ihrem distalen Ende mit einer weiteren Mikrowendel 7 mit mittlerem Durchmesser verbunden ist. Diese Mikrowendel 7 mittleren Durchmessers fügt sich teilweise um die erste Mikrowendel 6 und ist mit dieser ebenfalls schweißtechnisch verbunden. Um die zweite Mikrowendel 7 fügt sich schließlich die am längsten dimensionierte dritte Mikrowendel 8 mit größtem Durchmesser, in deren Inneren der aus einer Nickel-Titanlegierung bestehende Sicherungsdaht 9 verläuft.

Der Draht 9 ist an seinen beiden Enden durch artfremdes Verschweißen jeweils mit einem aus einer Platin-Iridiumlegierung bestehenden Verbindungsmittel 10 befestigt. Die beiden, an den Enden des Sicherungsdrahtes 9 angebrachten Verbindungsmittel sind ebenfalls als Mikrowendeln 10'/10" ausgebildet, die jeweils mit der proximalen bzw. distalen zweiten Mikrowendel 7 eines jeden Segmentes fest verschweißt sind. Der Sicherungsdraht ist so dimensioniert, daß seine Länge größer ist als die Länge des Segmentes 5, welches er durchspannt. Aufgrund dieser Ausbildung ist die als Mikrowendel ausgebildete Occlusionswendel 3 besonders flexibel und gleichzeitig biege- bzw. torsionsstabil.

Der das Sicherungsmittel 9 bildende Draht weist einen mittleren Durchmesser von ca. 0,03 bis 0,05 mm auf und verjüngt sich zu seinem proximalen Ende (d. h. zum Führungsdraht hin), so daß die jeweils ersten in ein Aneurysma eingeschobenen Bereiche der Occlusionswendel stabile Strukturen ausbilden, welche von den nachfolgenden, proximalen Bereichen nur noch ausgefüllt werden, ohne daß diese proximalen Bereiche eine zu große Kraft auf das sich bereits im Füllungsprozeß befindende Aneurysma ausüben. Diese Ausführungsform minimiert das Risiko der Wandruptur durch die zuletzt in das Aneurysma eingebrachten proximalen Bereiche der Occlusionswendel 3. Die distale Spitze der Occlusionswendel 11 ist abgerundet, um die Gefahr einer Traumatisierung des Aneurysmas zu minimieren. Im Inneren ist die Spitze 11 mit der distalen, als Verbindungsmittel dienenden Mikrowendel10 schweißtechnisch fest verbunden, so daß auch im Falle des Abbrechens oder Abreißens der Spitze 11 und angrenzender Bereiche der Occlusionswendel 3 von dem proximalen Rest der Occlusionswendel 3 die Spitze 11 nicht in den Blutstrom gelangt und dort möglicherweise Embolien verursacht.

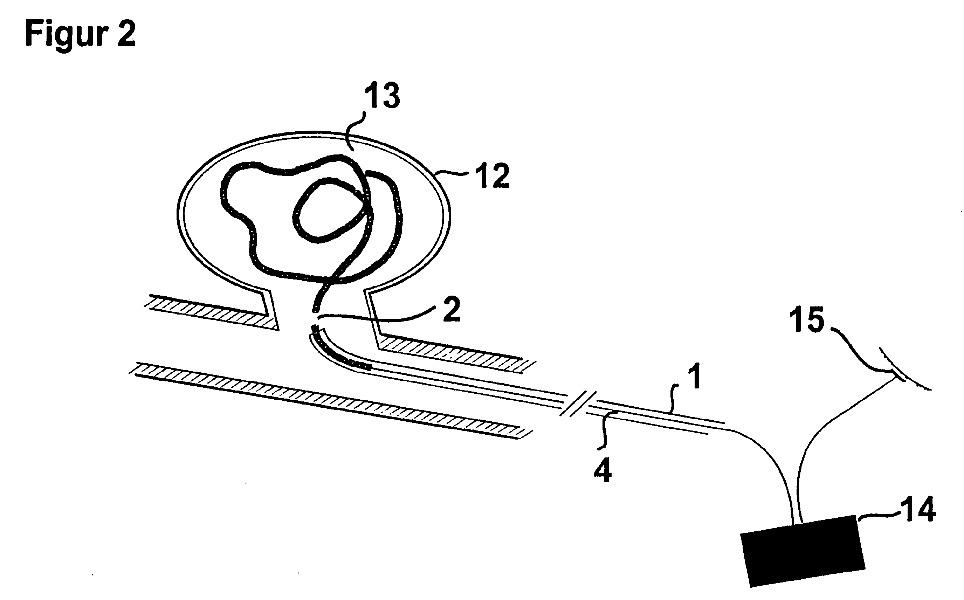

In der Figur 2 ist die vertikale Ansicht einer in einem Beerenaneurysma 12 positionierten Occlusionswendel 3 dargestellt. Durch die in Längsachse des Mikrokatheters 1 nach distal erfolgende Verschiebung der Führungshilfe 4 erfolgt die Einführung der Mikrowendel 3 in das Aneurysma 12, welche nach Verlassen des Mikrokatheters 1 sekundäre Windungen 13 ausbildet. Die Ausbildung der sekundären Windungen 13 wird durch den Sicherungsdraht 9 gewährleistet, welcher hier gleichzeitig zur Formgebung der Occlusionswendel 3 beiträgt. Dieser aus einer Titan-Nickellegierung bestehende Draht ist als Feder ausgebildet, die zudem zu einem übergeordneten, zwei oder dreidimensionalem Design vorgeformt ist.

Bei Unterbringung der Occlusionswendel im Katheter erfolgt eine spannungsinduzierte martensitische Transformation, da Sicherungsdraht das übergeordnete Design nicht einnehmen kann. Bei Herausschieben der Occlusionswendel aus dem Mikrokatheter kommt es zu einem Wegfall der mechanischen Spannung und einem leichten Anstieg der Umgebungstemperatur zur Körpertemperatur. Infolge dieser Einflüsse macht der Sicherungsdraht eine teilweise spannungs- und teilweise temperaturinduzierte Umwandlung durch und geht in die Austenit Phase über. Bei Einführung der Occlusionswendel in den Blutstrom erfolgt somit eine Kombination aus superelastischer und temperaturinduzierter Transformierung des Sicherungsdrahtes. Die Mikrostruktur des Werkstoffs des Sicherungsdrahtes macht dabei eine Transformation durch, der Sicherungsdraht hingegen nur eine Formänderung. Der Sicherungsdraht nimmt wieder das übergeordnete Design ein. Die Formänderung des Sicherungsdrahtes, führt dazu, daß die Occlusionswendel unter Krafteinwirkung des Sicherungsdrahtes ein vordefiniertes, zwei oder dreidimensionales Design einnimmt.

Durch die Längsverschieblichkeit von Führungsdraht 4 und Occlusionswendel 3 im Mikrokatheter 1 wird eine an das Volumen des jeweils auszufüllenden Hohlraumes individuell angepaßte Länge der Occlusionswendel 3 in diesem eingebracht. Anschließend wird mit Hilfe der Spannungsquelle 14, der auf der Körperoberfläche positionierten Kathode 15 und der als Anode dienenden, im zu occludierenden Aneurysma 12 positionierten Occlusionswendel 3 eine Spannung über einen Zeitraum von 0,1 bis 20 min angelegt. Dadurch wird die elektrolytische Abtrennung des sich im Blut befindenden Teils der Occlusionswendel an der dem distalen Katheterende nächstliegenden elektrolytisch korrodierbaren Stelle 2 aufgelöst. Figur 2 stellt eine Occlusionswendel dar, deren dem distalen Ende des Mikrokatheters 1 nächstliegende elektrolytisch korrodierbare Stelle 2 bereits elektrolytisch korrodiert wurde.

## Patentansprüche

1. Medizinisches Implantat mit einer Occlusionswendel (3) und mindestens einem in der Occlusionswendel (3) befestigten, als flexibles Längenelement ausgebildetem Sicherungsmittel (9), welches die Länge der Occlusionswendel (3) zumindest in einem Teilbereich durchläuft, wobei das Sicherungsmittel (9) ein Material mit Formgedächtniseigenschaften enthält und mittels Mikrowendeln (7, 10', 10") in der Occlusionswendel (3) befestigt ist, welche mit dem Sicherungsmittel (9) und der Occlusionswendel (3) fest verbunden sind, **dadurch gekennzeichnet, dass** die Mikrowendeln (7, 10', 10") aus demselben Material gefertigt sind wie die Occlusionswendel (3).

2. Vorrichtung zur Implantation von Occlusionswendeln (3) in Körperhohlräumen oder Blutgefäßen mit einem Katheter (1), einem in dem Katheter (1) in Längsrichtung verschiebbaren medizinischen Implantat mit einer Occlusionswendel (3) gemäß Anspruch 1.

3. Vorrichtung gemäß Ansprüch 2, **dadurch gekennzeichnet, daß** das Sicherungsmittel (9) im wesentlichen aus einem Material mit Formgedächtniseigenschaften besteht.

4. Vorrichtung gemäß einem der Ansprüche 2 oder 3, **dadurch gekennzeichnet, daß** das Sicherungsmittel (9) länger dimensioniert ist als der Teilbereich der Occlusionswendel (3), über den es sich erstreckt.

5. Vorrichtung gemäß einem der vorstehenden Ansprüche, **dadurch gekennzeichnet, daß** das Material eine metallische Legierung mit der Fähigkeit ist, eine temperaturinduzierte martensitische Transformation zu durchlaufen.

6. Vorrichtung gemäß einem der vorstehenden Ansprüche, **dadurch gekennzeichnet, daß** die metallische Legierung die Fähigkeit aufweist, eine spannungsinduzierte martensitische Transformation zu durchlaufen.

7. Vorrichtung gemäß einem der Ansprüche 5 oder 6, **dadurch gekennzeichnet, daß** die Legierung eine Titan und Nickel enthaltende Legierung, eine Eisenbasis- oder Kupferbasislegierung ist.

8. Vorrichtung gemäß einem der Ansprüche 5 bis 7, **dadurch gekennzeichnet, daß** das Sicherungsmittel (9) ein Draht ist.

9. Vorrichtung gemäß einem der vorstehenden Ansprüche, **dadurch gekennzeichnet, daß** sich das Sicherungsmittel (9) bis zum distalen Spitzenabschnitt der Occlusionswendel (3) erstreckt und sein eines Ende mit diesem fest verbunden ist.

10. Vorrichtung gemäß einem vorstehenden Ansprüche, **dadurch gekennzeichnet, daß** sich der die Occlusionswendel (3) bildende Draht zu seinem proximalen und/oder distalen Ende hin verjüngt.

11. Vorrichtung gemäß einem der vorstehenden Ansprüche, **dadurch gekennzeichnet, daß** sich das Sicherungsmittel (9) zu seinem proximalen und/oder distalen Ende hin verjüngt.

12. Vorrichtung gemäß einem der vorstehenden Ansprüche, **dadurch gekennzeichnet, daß** die Verbindung der Mikrowendeln (6, 7, 10', 10") mit der Occlusionswendel (3) und/oder dem Sicherungsmittel (9) durch Verschweißen, Verlöten, Verkleben oder mechanische, insbesondere kraft- und/oder formschlüssige Verbindungen erfolgt.

13. Vorrichtung gemäß einem der vorstehenden Ansprüche, **dadurch gekennzeichnet, daß** das die Mikrowendeln (7, 10', 10") und die Occlusionswendel (3) bildende Material Platin oder eine Platinlegierung ist.

14. Vorrichtung gemäß einem der vorstehenden Ansprüche, **dadurch gekennzeichnet, daß** die Occlusionswendel (3) als Mikrowendel (8) ausgebildet ist.

15. Vorrichtung gemäß einem der vorstehenden Ansprüche, **dadurch gekennzeichnet, daß** das Sicherungsmittel (9) vorgeformt ist.

16. Vorrichtung gemäß Anspruch 15, **dadurch gekennzeichnet, daß** das Sicherungsmittel (9) zu einer Wendel oder Feder und besonders bevorzugt zu einer helikalen Wendel oder einer Spiralfeder vorgeformt ist.

17. Vorrichtung gemäß einem der vorstehenden Ansprüche, **dadurch gekennzeichnet, daß** die Occlusionswendel (3) zu einem übergeordneten Design, vorzugsweise zu sekundären Windungen (13) oder einem käfigartigem Design vorgeformt ist.

18. Vorrichtung gemäß einem der Ansprüche 15 bis 17, **dadurch gekennzeichnet, daß** das vorgeformte Sicherungsmittel (9) die Fähigkeit aufweist, bei Körpertemperatur eine temperaturinduzierte martensitische Transformation durchzulaufen.

19. Vorrichtung gemäß Anspruch einem der Ansprüche 15 bis 18, **dadurch gekennzeichnet, daß** das vorgeformte Sicherungsmittel (9) innerhalb des Katheters gestreckt ist und in martensitischer Phase vorliegt und bei Ausbringung in das Blutgefäßsystem bzw. das Aneurysma (12) eine zumindest teilweise spannungsinduzierte martensitische Transformation durchmacht und seine alte Form einnimmt.

20. Vorrichtung gemäß einem der vorstehenden Ansprüche, **gekennzeichnet durch** eine Occlusionswendel (3) mit einer oder mehreren, in Abständen voneinander angeordneten elektrolytisch korrodierbaren Stellen (2) sowie einem elektrisch isolierenden Katheter (1), einer Spannungsquelle (14) und einer Kathode (15), die es im Kontakt mit einer Körperflüssigkeit erlaubt, eine oder mehrere variabel dimensionierbare Längen der Occlusionswendel (3) durch elektrolytische Prozesse abzutrennen.

21. Vorrichtung gemäß Anspruch 20, **dadurch gekennzeichnet, daß** die Occlusionswendel (3) mehrere elektrolytisch korrodierbare Stellen (2) aufweist, wobei in jedem zwischen diesen Stellen gelegenen Segment der Occlusionswendel (3) ein Sicherungsmittel (9) angeordnet ist, welches sich vorzugsweise jeweils von einem zum anderen Ende jedes Segmentes erstreckt.

22. Vorrichtung gemäß Anspruch 21 **dadurch gekennzeichnet, daß** jedes Segment der Occlusionswendel (3) von mindestens zwei, vorzugsweise drei, ineinandergefügten Mikrowendeln (6, 7, 8) unterschiedlichen Durchmessers gebildet wird, wobei die jeweils proximal bzw. distal an die elektrolytisch korrodierbare Stelle (2) angrenzende Wendel mit dieser fest verbunden ist.

23. Vorrichtung gemäß einem der vorstehenden Ansprüche, **dadurch gekennzeichnet, daß** sich proximal an die Occlusionswendel (3) eine als Führungsdraht (4) ausgebildete Einführhilfe anschließt.

24. Vorrichtung gemäß einem der vorstehenden Ansprüche zur Verwendung in human- oder tiermedizinischen Verfahren, vorzugsweise bei der Thrombosierung.

## Claims

1. Medical implant comprising an occlusion coil (3) and at least one longitudinally flexible safety element (9) attached in the occlusion coil (3) and extending through at least a part of the length of the occlusion coil (3), wherein the safety element (9) comprises a material with shape memory properties and is attached in the occlusion coil (3) by means of microcoils (7, 10', 10") that are rigidly connected with the safety element (9) and the occlusion coil (3), **characterized in that** the microcoils (7, 10', 10") are produced from the same material as the occlusion coil (3).

2. Device for implanting occlusion coils (3) in body cavities or blood vessels with a catheter (1) and a medical implant with an occlusion coil (3) according to claim 1, wherein the medical implant can be moved within the catheter (1) in the longitudinal direction.

3. Device according to claim 2, **characterized in that** the safety element (9) essentially consists of a material with shape memory properties.

4. Device according to claim 2 or 3, **characterized in that** the safety element (9) is sized to be longer than the part of the occlusion coil (3) over which it extends.

5. Device according to any of the preceding claims, **characterized in that** the material is a metal alloy with the capacity of undergoing temperature-induced martensite transformation.

6. Device according to any of the preceding claims, **characterized in that** the metal alloy has the capacity of undergoing stress-induced martensite transformation.

7. Device according to claim 5 or 6, **characterized in that** the alloy is an alloy containing titanium and nickel, an iron-based alloy or a copper-based alloy.

8. Device according to any of claims 5 to 7, **characterized in that** the safety element (9) is a wire.

9. Device according to any of the preceding claims, **characterized in that** the safety element (9) extends to the distal tip segment of the occlusion coil (3) and that its one end is rigidly connected with the distal tip segment.

10. Device according to any of the preceding claims, **characterized in that** the wire that forms the occlusion coil (3) becomes thinner towards its proximal and/or distal end.

11. Device according to any of the preceding claims, **characterized in that** the safety element (9) becomes thinner towards its proximal and/or distal end.

12. Device according to any of the preceding claims, **characterized in that** the connection between the microcoils (6, 7, 10', 10") and the occlusion coil (3) and/or the safety element (9) is brought about by welding, soldering, gluing, or mechanical connections, particularly non-positive-lock and/or positive-lock connections.

13. Device according to any of the preceding claims, **characterized in that** the material forming the microcoils (7, 10', 10") and the occlusion coil (3) is platinum or a platinum alloy.

14. Device according to any of the preceding claims, **characterized in that** the occlusion coil (3) is structured as a microcoil (8).

15. Device according to any of the preceding claims, **characterized in that** the safety element (9) is preformed.

16. Device according to claim 15, **characterized in that** the safety element (9) is preformed to a coil or spring, and particularly preferably, to a helical coil or a coil spring.

17. Device according to any of the preceding claims, **characterized in that** the occlusion coil (3) is preformed to produce an overriding design, preferably secondary coils (13) or a cage-like design.

18. Device according to any of claims 15 to 17, **characterized in that** the preformed safety element (9) demonstrates the capacity to pass through temperature-induced martensite transformation at body temperature.

19. Device according to any of claims 15 to 18, **characterized in that** the preformed safety element (9) is stretched within the catheter and is present in the martensite phase and undergoes at least partial stressed-induced martensite transformation when it is placed in the blood vessel system or the aneurysm (12), and assumes its old shape.

20. Device according to any of the preceding claims, **characterized by** an occlusion coil (3) with one or more electrolytically corrodable locations (2) arranged at a distance from one another, as well as with an electrically insulating catheter (1), a voltage source (14), and a cathode (15) that makes it possible to separate one or more variably sizeable lengths of the occlusion coil (3) by means of electrolytic processes, upon contact with the body fluid.

21. Device according to claim 20, **characterized in that** the occlusion coil (3) has several electrolytically corrodable locations (2), wherein a safety element (9) is arranged in each segment of the occlusion coil (3) located between these locations, preferably extending from one end to the other of each segment, in each instance.

22. Device according to claim 21, **characterized in that** each segment of the occlusion coil (3) is formed by at least two, preferably three microcoils (6, 7, 8) with different diameters, inserted one inside the other, where the coil that is adjacent to the electrolytically corrodable location (2), proximally or distally, in each instance, is rigidly connected with the latter.

23. Device according to any of the preceding claims, **characterized in that** an insertion aid structured as a guide wire (4) follows in the proximal direction of the occlusion coil (3).

24. Device according to any of the preceding claims for use in human medicine or veterinary medicine procedures, preferably in thrombotization.

## Revendications

1. Implant médical avec une hélice d'occlusion **(3)** et au moins un moyen d'immobilisation **(9)** conformé en élément de longueur flexible et fixé dans l'hélice d'occlusion **(3),** qui traverse la longueur de l'hélice d'occlusion **(3)** sur au moins une partie, le moyen d'immobilisation **(9)** contenant un matériau à propriétés de mémoire de forme et étant fixé dans l'hélice d'occlusion **(3)** au moyen de micro-hélices **(7, 10', 10"),** qui sont reliées fixement au moyen d'immobilisation **(9)** et à l'hélice d'occlusion **(3), caractérisé en ce que** les micro-hélices **(7, 10', 10")** sont fabriquées dans le même matériau que l'hélice d'occlusion **(3).**

2. Dispositif pour implanter des hélices d'occlusion **(3)** dans des cavités corporelles ou des vaisseaux sanguins avec un cathéter **(1),** avec un implant médical selon la revendication 1 comprenant une hélice d'occlusion **(3)** et pouvant être déplacé dans la direction longitudinale dans le cathéter **(1).**

3. Dispositif selon la revendication 2, **caractérisé en ce que** le moyen d'immobilisation **(9)** est constitué pour l'essentiel d'un matériau ayant des propriétés de mémoire de forme.

4. Dispositif selon la revendication 2 ou 3, **caractérisé en ce que** le moyen d'immobilisation **(9)** est réalisé plus long que la partie de l'hélice d'occlusion **(3)** sur laquelle il s'étend.

5. Dispositif selon l'une des revendications précédentes, **caractérisé en ce que** le matériau est un alliage métallique présentant la capacité de subir une transformation martensitique induite par la température.

6. Dispositif selon l'une des revendications précédentes, **caractérisé en ce que** l'alliage métallique présente la capacité de subir une transformation martensitique induite par des contraintes.

7. Dispositif selon l'une des revendications 5 ou 6, **caractérisé en ce que** l'alliage est un alliage contenant du titane et du nickel, un alliage à base de fer ou un alliage à base de cuivre.

8. Dispositif selon l'une des revendications 5 à 7, **caractérisé en ce que** le moyen d'immobilisation **(9)** est un fil.

9. Dispositif selon l'une des revendications précédentes, **caractérisé en ce que** le moyen d'immobilisation **(9)** s'étend jusqu'à la section de pointe distale de l'hélice d'occlusion **(3)** et sa première extrémité est reliée fixement à celle-ci.

10. Dispositif selon l'une des revendications précédentes, **caractérisé en ce que** le fil formant l'hélice d'occlusion **(3)** est effilé en direction de son extrémité proximale et/ou distale.

11. Dispositif selon l'une des revendications précédentes, **caractérisé en ce que** le moyen d'immobilisation **(9)** est effilé en direction de son extrémité proximale et/ou distale.

12. Dispositif selon l'une des revendications précédentes, **caractérisé en ce que** la liaison entre les micro-hélices **(6, 7, 10', 10")** et l'hélice d'occlusion **(3)** et/ou le moyen d'immobilisation **(9)** s'effectue par soudage, brasage, collage ou par une liaison mécanique, en particulier par adhérence et/ou par engagement positif.

13. Dispositif selon l'une des revendications précédentes, **caractérisé en ce que** le matériau formant les micro-hélices **(7, 10', 10")** et l'hélice d'occlusion **(3)** est du platine ou un alliage de platine.

14. Dispositif selon l'une des revendications précédentes, **caractérisé en ce que** l'hélice d'occlusion **(3)** est conformée en micro-hélice **(8).**

15. Dispositif selon l'une des revendications précédentes, **caractérisé en ce que** le moyen d'immobilisation **(9)** est préformé.

16. Dispositif selon la revendication 15, **caractérisé en ce que** le moyen d'immobilisation **(9)** est préformé en une hélice ou un ressort et, de manière particulièrement préférée, en une hélice verticale ou un ressort en spirale.

17. Dispositif selon l'une des revendications précédentes, **caractérisé en ce que** l'hélice d'occlusion **(3)** est préformée en une forme de niveau supérieur, de préférence en des hélices secondaires **(13)** ou une forme de cage.

18. Dispositif selon l'une des revendications 15 à 17, **caractérisé en ce que** le moyen d'immobilisation **(9)** préformé présente la capacité de subir, à la température du corps, une transformation martensitique induite par la température.

19. Dispositif selon l'une des revendications 15 à 18, **caractérisé en ce que** le moyen d'immobilisation **(9)** préformé est étiré à l'intérieur du cathéter et est présent en phase martensitique et, après introduction dans le système de vaisseaux resp. l'anévrysme **(12),** il subit au moins partiellement une transformation martensitique induite par contraintes et prend son ancienne forme.

20. Dispositif selon l'une des revendications précédentes, **caractérisé par** une hélice d'occlusion **(3)** avec une ou plusieurs zones **(2)** corrodables électrolytiquement et placées à distance les unes des autres, ainsi qu'avec un cathéter isolant électrique **(1),** une source de tension **(14)** et une cathode **(15)** qui, au contact d'un liquide du corps, permet de séparer une ou plusieurs longueurs de dimensions variables de l'hélice d'occlusion **(3)** par des processus électrolytiques.

21. Dispositif selon la revendication 20, **caractérisé en ce que** l'hélice d'occlusion **(3)** présente plusieurs zones **(2)** corrodables électrolytiquement, un moyen d'immobilisation **(9)** étant placé dans chaque segment de l'hélice d'occlusion **(3)** situé entre ces zones, moyen de sécurisation qui s'étend de préférence d'une extrémité à l'autre de chaque segment.

22. Dispositif selon la revendication 21, **caractérisé en ce que** chaque segment de l'hélice d'occlusion **(3)** est formé par au moins deux, de préférence trois micro-hélices **(6, 7, 8)** de diamètres différents insérées les unes dans les autres, l'hélice voisine à chaque fois distalement ou proximalement de la zone **(2)** corrodable électrolytiquement étant reliée fixement à elle.

23. Dispositif selon l'une des revendications précédentes, **caractérisé en ce qu'**un auxiliaire d'introduction conformé en fil de guidage **(4)** fait suite de façon proximale à l'hélice d'occlusion **(3).**

24. Dispositif selon l'une des revendications précédentes, destiné à une utilisation dans des procédures médicales humaines ou animales, de préférence dans le traitement de thromboses.
